# EUROPÄISCHE PATENTSCHRIFT

(11) **EP 0 907 638 B1**
(45) Veröffentlichungstag und Bekanntmachung des Hinweises auf die Patenterteilung: **27.11.2002**
(21) Anmeldenummer: 97928229.0
(22) Anmeldetag: 18.06.1997
(51) Int. Cl.: C07C 311/58, C07C 311/59, C07C 311/39

(54) **4-JOD-2- N-(N-ALKYL-AMINOCARBONYL)-AMINOSULFONYL]-BENZOESÄUREMETHYLESTER UND -DERIVATE UND VERFAHREN ZU DEREN HERSTELLUNG**
4-JOD-2 N-(N-ALKYL-AMINOCARBONYL)-AMINOSULFONYL]-BENZOIC ACID METHYL ESTER AND DERIVATIVED THEREOF AND METHOD FOR THEIR PRODUCTION
METHYLESTER DE L'ACIDE 4-IODO-2- N-(N-ALKYL-AMINOCARBONYL)-AMINOSULFONYL]-BENZOIQUE ET SES DERIVES, ET SON PROCEDE DE PRODUCTION

(30) Priorität: 28.06.1996 DE 19625831
(43) Veröffentlichungstag der Anmeldung: 14.04.1999
(73) Patentinhaber: Bayer CropScience GmbH, 65929 Frankfurt/Main (DE)
(72) Erfinder: WILLMS, Lothar, D-65719 Hofheim (DE); KNORR, Harald, D-60529 Frankfurt am Main (DE)
(86) Internationale Anmeldenummer: EP9703170
(87) Internationale Veröffentlichungsnummer: WO98000396

(56) Entgegenhaltungen:
- WO-A-92/13845
- US-A- 4 383 113
- US-A- 4 566 898
- SEARLE ET AL.: "Stereochemistry of Diphenyls. XXX. Preparation and Resolution of 2,2'-Diodo-4,4'-dicarboxydiphenyl" JOURNAL OF AMERICAN CHEMICAL SOCIETY, Bd. 55, 1933, Seiten 1649-1654, XP002049313 in der Anmeldung erwähnt

## Beschreibung

Die Erfindung betrifft das technische Gebiet der Zwischenprodukte für die Herstellung von Wirkstoffen, insbesondere von herbiziden Sulfonylharnstoffen.

Es ist bekannt, daß man Harnstoffe der Formel Aryl-SO₂-NH-CO-NH-R, worin Aryl einen gegebenenfalls substituierten Arylrest und R einen Alkylrest bedeuten, im Prinzip mit Phosgen zu entsprechenden Isocyanaten der Formel Aryl-SO₂-NCO umsetzen kann, welche ihrerseits zur Herstellung von herbizid wirksamen Sulfonylharnstoffen eingesetzt werden können (EP-A-0584043; US-A-4566898). Um die allgemeine Methode auf die Herstellung von Sulfonylharnstoffen in der Reihe Aryl = 2-Carbomethoxy-5-jod-phen-1-yl anwenden zu können, welche wertvolle herbizide Sulfonylharnstoffe darstellen (WO-A-92/13845), stellte sich die Aufgabe, Verbindungen der Formel (I) bereitzustellen, 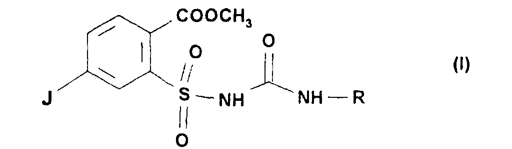 worin R = Alkyl oder Cycloalkyl bedeutet, wobei jeder der beiden letztgenannten Reste unsubstituiert oder substituiert ist. Vorzugsweise bestand die Aufgabe darin, ein effektives Verfahren zur Herstellung der Verbindungen der Formel (I) bereitzustellen.

Überraschenderweise gelingt es, wie nachstehend näher erläutert, die Verbindungen der Formel (I) ausgehend von Verbindungen der Formel (II) über die Zwischenstufen (III) und (IV) in sehr guten Ausbeuten zu erhalten. Mit der Erfindung wird somit eine effektive Herstellung von herbiziden Sulfonylharnstoffen und anderen Wirkstoffen ermöglicht.

Gegenstand der Erfindung ist ein Verfahren zur Herstellung von N-substituierten 4-Jod-2-[N-(aminocarbonyl)-aminosulfonyl]-benzoesäuremethylestern der Formel (I), 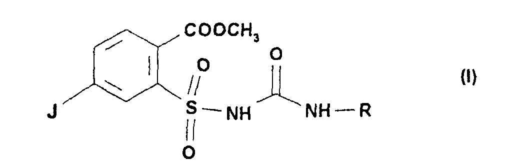 worin R = Alkyl oder Cycloalkyl, wobei jeder der beiden letztgenannten Reste unsubstituiert oder substituiert ist, bedeutet und
vorzugsweise R = (C₁-C₁₂)Alkyl, das unsubstituiert oder durch einen oder mehrere Reste aus der Gruppe Halogen, (C₁-C₆)Alkoxy, (C₁-C₆)Alkylthio, (C₁-C₆)Haloalkoxy und gegebenenfalls substituiertes Phenyl substituiert ist, oder (C₃-C₁₂)Cycloalkyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₆)Alkylthio substituiert ist, bedeutet,
dadurch gekennzeichnet, daß man
(a) eine Verbindung der Formel (II) 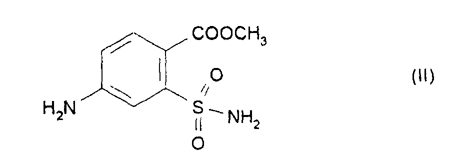 durch Diazotierung in Gegenwart einer Säure H⁺X⁻, wobei X⁻ ein Äquivalent eines Anions, beispielsweise X⁻ = Cl⁻, J⁻ oder HSO₄⁻, bedeutet, zu einer Verbindung (Diazoniumsalz) der Formel (III), 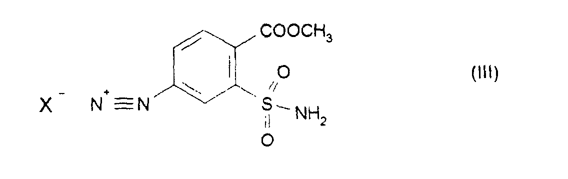 worin X⁻ die Bedeutung wie in der Säure H⁺X⁻ hat, umsetzt und
(b) die Verbindung der Formel (III), nach deren Isolierung oder vorzugsweise ohne Isolierung, in Gegenwart von Jodidionen zu einer Verbindung der Formel (IV) 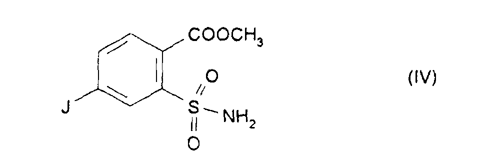 umsetzt und
(c) die Verbindung der Formel (IV) mit einem Isocyanat der Formel (V),

   R-N=C=O (V)

   worin R wie in Formel (I) definiert ist, zur Verbindung der Formel (I) umsetzt.
Weiterhin sind Gegenstand der Erfindung auch Teilschritte des Verfahrens.

In der Formel (I) und den im folgenden verwendeten allgemeinen Formeln können die Reste Alkyl, Alkoxy, Haloalkyl, Haloalkoxy und Alkylthio sowie die entsprechenden substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 4 C-Atomen bevorzugt. Alkylreste, auch in den zusammengesetzten Bedeutungen wie Alkoxy, Haloalkyl usw. bedeuten, z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyle, 1-Methylhexyl und 1,4-Dimethylpentyl.
Cycloalkyl bedeutet ein carbocyclisches gesättigtes Ringsystem, beispielsweise mit 3 bis 8 Ringatomen, z.B. Cyclopropyl, Cyclobutyl, Cyclopentyl, Cyclohexyl usw. Halogen bedeutet beispielsweise Fluor, Chlor, Brom oder lod. Haloalkyl, -alkenyl und-alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl₂, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Sind Substitutionen definiert durch "einen oder mehrere Reste aus einer Gruppe von Resten" beinhaltet dies sowohl die Substitution durch einen oder mehrere gleiche Reste als auch die einfache oder mehrfache Substitution durch unterschiedliche Reste.
Gegebenenfalls substituiertes Phenyl ist vorzugsweise Phenyl, das unsubstituiert oder ein- oder mehrfach durch gleiche oder verschiedene Reste aus der Gruppe Halogen, Alkyl, Haloalkyl, Alkoxy, Haloalkoxy, Alkylthio, Hydroxy, Amino, Nitro, Cyano, Azido, Alkoxycarbonyl, Alkylcarbonyl, Formyl, Carbamoyl, Mono- und Dialkylaminocarbonyl, substituiertes Amino, wie Acylamino, Mono- oder Dialkylamino, und Alkylsulfinyl, Haloalkylsulfinyl, Alkylsulfonyl, Haloalkylsulfonyl vorzugsweise bis zu dreifach durch gleiche oder verschiedene Reste aus der Gruppe Halogen, (C₁-C₄)-Alkyl, (C₁-C₄)-Alkoxy, (C₁-C₄)-Halogenalkyl, (C₁-C₄)-Halogenalkoxy, CN und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome, die in der allgemeinen Formel (I) nicht gesondert angegeben sind. Die durch ihre spezifische Raumform definierten möglichen Stereoisomeren, wie Enantiomere, Diastereomere sind alle von der Formel (I) umfaßt und können nach üblichen Methoden aus Gemischen der Stereoisomeren erhalten oder auch durch stereoselektiven Reaktionen in Kombination mit dem Einsatz von stereochemisch reinen Ausgangsstoffen hergestellt werden. Formel (I) umfaßt auch Tautomere der bezeichneten Verbindungen, soweit sie durch Protonenwanderung entstehen und soweit sie chemisch stabil sind.

Die Verbindungen der Formel (I) können Salze bilden, bei denen der Wasserstoff der -SO₂-NH-Gruppe oder auch andere acide Wasserstoffatome durch ein für die Landwirtschaft geeignetes Kation ersetzt wird. Diese Salze sind beispielsweise Metallsalze; vorzugsweise Alkali- oder Erdalkalisalze, insbesondere Natrium- und Kaliumsalze, oder auch Ammoniumsalze oder Salze mit organischen Aminen, Ebenso kann Salzbildung durch Anlagerung einer Säure an basische Gruppen, wie z.B. Amino und Alkylamino, erfolgen. Geeignete Säuren hierfür sind starke anorganische und organische Säuren, beispielsweise HCI, HBr, H₂SO₄ oder HNO₃.

Die Verbindung der Formel (II) ist bekannt (vgl. EP-A-382436, EP-A-382437) oder kann nach Standardverfahren aus einfachen kommerziell erhältlichen Verbindungen hergestellt werden (vgl. z. B. Herstellung des analogen Isopropylesters in J. Org. Chem. 27 (1962) 2177 ff.).

Die Diazotierung der Verbindung der Formel (II) nach Verfahrensschritt (a) kann unter an sich üblichen Bedingungen für Diazotierungsreaktionen durchgeführt werden. Beispielsweise wird die Diazotierung der Verbindung der Formel (II) in Gegenwart der Säure H⁺X⁻, wobei X⁻ vorzugsweise Cl⁻, J⁻ oder HSO₄⁻ ist, in wäßriger Lösung und gegebenenfalls vorzugsweise unter Zusatz eines unter den Reaktionsbedingungen inerten organischen Lösungsmittels mit einem Nitrit durchgeführt. Beispielsweise diazotiert man mit einem Alkalimetallnitrit wie NaNO₂ (Natriumnitrit) in Mengen von 1,0 - 1,2 Mol Nitrit, vorzugsweise 1,01-1,05 Mol Nitrit, pro Mol der Verbindung der Formel (II).

Als Säuren für die Diazotierung eignen sich Mineralsäuren oder starke organische Säuren. Bevorzugt sind Halogenwasserstoffsäuren, wie Salzsäure oder Jodwasserstoffsäure, oder Schwefelsäure. Die Säure H⁺X⁻ wird wie üblich im Überschuß, bezogen auf die stöchiometrische Menge, eingesetzt; im Falle von n-wertigen Säuren mit n größer 1 wie Schwefelsäure (n=2) ist die stöchiometrische Menge für eine einwertige Säure durch n zu teilen, um die stöchiometrische Menge für die n-wertige Säure zu erhalten. Beispielsweise setzt man eine einbasige Säure in einer Menge von 5 bis 15 Mol pro Mol Verbindung der Formel (II), vorzugsweise 8 bis 12 Mol H⁺X⁻ pro Mol Verbindung der Formel (II) ein.

Das Lösungsmittel bei der Diazotierung ist Wasser oder eine Mischung aus Wasser und einem unter den Reaktionsbedingungen inerten organischen Lösungsmittel, z.B. aus der Gruppe enthaltend
- gegebenenfalls substituierte aromatische Kohlenwasserstoffe, wie Toluol, Chlorbenzol, Dichlorbenzole, Chlortoluole oder Xylole,
- halogenierte aliphatische Kohlenwasserstoffe wie Dichlormethan und
- Ether wie Diethylether, Dioxan und Tetrahydrofuran.

Die geeignete Temperatur für die Diazotierung zur Verbindung der Formel (III) kann in Vorversuchen leicht bestimmt werden und ist in der Regel im Temperaturbereich von -5 bis 50 °C, vorzugsweise von 10 bis 20 °C, insbesondere von 15 bis 20 °C.

Die Diazoniumsalze der Verbindungen der Formel (III) können nach übliche Bedingungen isoliert werden, wobei jedoch die relativ geringe Stabilität der Diazoniumgruppe zu beachten ist; vorzugsweise aber werden die Verbindungen (III) ohne Zwischenisolierung für die Herstellung der Verbindungen der Formel (IV) verwendet.

Die Diazoniumsalze der Formel (III), vorzugsweise mit X⁻ = J⁻ (Jodid-Anion), sind neu und ebenfalls Gegenstand der Erfindung.

Die Umsetzung der Verbindung (III) zur Verbindung der Formel (IV) nach Verfahrensstufe (b) wird im gleichen wäßrigen oder wäßrig-organischen Lösungsmittel oder Lösungsmittelgemisch wie in Verfahrensstufe (a) oder in einem analogen modifizierten Lösungsmittel(gemisch) durchgeführt. Bei der Umsetzung wird die Diazoniumgruppe durch das Jodatom ausgetauscht, weshalb Jodidionen im Reaktionsgemisch benötigt werden. Letztere können im Fall vom X⁻ = J⁻ vom Anion des Diazoniumsalzes selbst stammen.
Wenn X⁻ vom Jodid-Anion verschieden und beispielsweise Cl⁻ ist, muß Jodid in anderer Form, z. B. als Alkalimetalljodid wie Natriumjodid oder Kaliumjodid zugesetzt werden. Die Menge beträgt dabei beispielsweise 1,1 bis 1,5 Mol Jodid pro Mol der Verbindung der Formel (III). Alternativ kann das Jodid bereits in der Vorstufe (a) der Verbindung der Formel (II) zugegeben werden.

Die Bildung der Verbindung der Formel (IV) aus der Verbindung der Formel (III) kann in der Regel im Temperaturbereich von 10 bis 40 °C, vorzugsweise von 15 bis 30 °C erfolgen.

Die Herstellung der Verbindung der Formel (IV) geschieht beispielsweise derart, daß man die Verbindung der Formel (II) in wäßriger Salzsäure vorlegt und mit katalytischen Mengen Toluol gegebenenfalls zusammen mit einem Entschäumer versetzt. Bei 15 bis 20 °C fügt man langsam eine wäßrige Lösung von NaNO₂ zu, rührt nach und zerstört den Nitritüberschuß mit Amidosulfonsäure. Das erhaltene Diazoniumsalz der Formel (III) (wobei X⁻ = Cl⁻ ist) wird als wäßrige Lösung zu einer wäßrigen Lösung von Kaliumjodid bei 15 bis 20 °C zugegeben. Man rührt nach und filtriert dann den Niederschlag, wäscht mit Bisulfit (z.B. NaHSO₃) jodidfrei und trocknet.

Überraschenderweise gelingt die Herstellung des Jodphenylsulfonamids (IV) über das Diazoniumsalze (III) in sehr guten Ausbeuten. Ausbeuten von weit über 90% d.Th. können erreicht werden.
Derartig hohe Ausbeuten sind nicht zu erwarten gewesen, wenn man bekannte Ausbeuten vergleichbarer Reaktionen berücksichtigt hat. Gemäß J. Am. Chem. Soc. 55 (1933) 1652 ergibt die Diazotierung von 4-Amino-benzoesäure-ethylester mit Natriumnitrit und Salzsäure und anschließender Reaktion unter Zusatz von Kaliumjodid 4-Jod-benzoesäure-ethylester in einer Ausbeute von nur 68,5% d. Th.; im Vergleich dazu verläuft die Reaktion mit Verbindungen der Formel (III), welche durch die Sulfonamidgruppe eine zusätzliche funktionelle Gruppe aufweisen, überraschend günstig.

Die Jodphenylverbindung der Formel (IV) selbst konnte bislang nur in unzureichender Ausbeute dargestellt werden. So gelangt man ausgehend von 4-Jod-2-chlorsulfonyl-benzoesäuremethylester in Gegenwart von Ammoniak nur in ca. 75 % Ausbeute zur Verbindung der Formel (IV) (siehe WO 92/13845, Bsp. 6, Seite 26); bei diesem Typ von Umsetzungen sind häufig Ausbeuteverluste durch Ringschluß zum Saccharinderivat beobachtet worden. Nach dem erfindungsgemäßen Verfahren fällt jedoch die Verbindung der Formel (IV) in sehr hoher Ausbeute und ohne das unerwünschte Jodsaccharin an.

Nach Verfahrensstufe (c) wird die Verbindung der Formel (IV) mit einem Isocyanat der Formel R-N=C=O (V) zu einer Verbindung der Formel (I) umgesetzt. Die Umsetzung erfolgt vorzugsweise unter der katalytischen Wirkung einer Base und in einem inerten Lösungsmittel, wobei die für die Verfahrenstufen (a) und (b) genannten zugesetzten organischen Lösungsmittel hier auch in Frage kommen; andere organische Lösungsmittel sind auch verwendbar, wobei geeignete Lösungsmittel in Vorversuchen durch Vergleich leicht zu finden sind.

Als Lösungsmittel bei der Verfahrensstufe (c) eignen sich vorzugsweise nicht wäßrige, unter den Reaktionsbedingungen inerte organische Lösungsmittel, z.B. aus der Gruppe enthaltend
- gegebenenfalls substituierte aromatische Kohlenwasserstoffe, wie Toluol, Chlorbenzol, Dichorbenzole, Chlortoluole oder Xylole,
- halogenierte aliphatische Kohlenwasserstoffe wie Dichlormethan,
- Ether wie Diethylether, Dioxan und Tetrahydrofuran und
- Ketone, wie Aceton.
Bevorzugt ist Chlorbenzol.

Als Basen eignen sich anorganische oder organische Basen, z. B. Carbonate wie K₂CO₃, Na₂CO₃, substituierte Amine wie Triethylamin, DBU (1,8-Diazabicyclo[5.4.0]undec-7-en) u.a.; bevorzugt ist eine Base wie K₂CO₃. Die Basen werden beispielsweise in Mengen von 1 Mol bis 1,1 Mol, vorzugsweise von 1,01 bis 1,05 Mol pro Mol Verbindung der Formel (IV) eingesetzt.

Die Mengen an Isocyanat der Formel (V) liegen beispielsweise bei 1,0 - 2,0 Mol, vorzugsweise bei 1,01 - 1,1 Mol, pro Mol Verbindung der Formel (IV). Als Isocyanate kommen vorzugsweise Methylisocyanat, Ethylisocyanat, n-Propylisocyanat, Isopropylisocyanat, n-Butylisocyanat, i-, sec- oder t-Butylisocyanat, n-Pentylisocyanat, n-Hexylisocyanat, Cyclohexylisocyanat, Dodecylisocyanat, vorzugsweise Isocyanate mit R gleich (C₄-C₆)Alkyl zum Einsatz.

Die Reaktionstemperatur in Verfahrensstufe (c) zur Herstellung von Verbindungen der Formel (I) wird vorzugsweise zweistufig geführt, wenn Lösungsmittel wie Toluol, Xylol oder Chlorbenzol verwendet werden. Zunächst hält man beispielsweise die Temperatur für 2 Stunden bei 55-60 °C, dann 5 bis 10 Stunden, vorzugsweise 6 bis 9 Stunden bei 90 °C. In Gegenwart von Lösungsmitteln wie Aceton wird beispielsweise zunächst bei Raumtemperatur unter Rühren, dann 4 bis 10 Stunden, vorzugsweise 5 bis 8 Stunden, unter Rückfluß und Rühren umgesetzt.

Die Herstellung der Verbindung der Formel (I) verläuft beispielsweise so, daß man das Sulfonamid der Formel (IV) mit K₂CO₃ und dem Isocyanat der Formel (V) in Chlorbenzol versetzt und zunächst bei 55 bis 60 °C, dann bei 90 °C reagieren läßt. Nach Abkühlen auf Raumtemperatur wird mit Wasser versetzt und mit 2n Salzsäure auf pH 1 bis 2 gestellt. Die organische Phase wird abgetrennt, säurefrei gewaschen und eingeengt. Man erhält als Rückstand die Verbindungen der Formel (I) in hohen Ausbeuten und sehr guten Reinheiten.

Die Verbindungen der Formel (I) sind bislang nur in unzureichender Ausbeute zugänglich gewesen. So gelangt man beispielsweise zum 2-[N-(N-Butylaminocarbonyl)-aminosulfonyl]-4-chlorbenzoesäure-isopropylester durch Umsetzung des 2-(Aminosulfonyl)-4-chlorbenzoesäureisopropylesters mit Butylisocyanat in 2-Butanon in nur 44 % d. Th. Ausbeute zum gewünschten Butylsulfonylharnstoff (US-A-4566898, Bsp. 5).

Überraschenderweise erhält man beim erfindungsgemäßen Verfahren jedoch die Verbindungen der Formel (I) in sehr hohen Ausbeuten. Bei dieser Methode entsteht wenig Abfall und die Volumenausbeuten sind hoch. Es war aus obengenannten Gründen daher nicht vorhersehbar, daß man die Verbindungen der Formel (I) ausgehend von der Verbindung der Formel (IV) oder der Verbindung der Formel (II) in so hoher Ausbeute bzw. Gesamtausbeute über mehrere Stufen erhalten würde.

In den folgenden Beispielen beziehen sich Mengenangaben auf das Gewicht, sofern nichts anderes speziell definiert ist.

### Beispiel 1

### 4-Jod-2-aminosulfonyl-benzoesäuremethylester (IV)

1000 g (4,34 Mol) 4-Amino-2-aminosulfonyl-benzoesäuremethylester und 3400 ml H₂O werden schnell mit 3700 ml konzentrierter Salzsäure (37 %ig) versetzt. Dazu gibt man 10 ml Toluol. Man kühlt auf 15 °C ab und läßt eine Lösung von 315 g NaNO₂ (4,56 Mol) in 1740 ml H₂O innerhalb von 1 Std. (Std. = Stunde) bei 15-20 °C zulaufen. Man rührt 1 Std. nach und zerstört den Nitritüberschuß mit Amidosulfonsäure. In einem zweiten Gefäß werden 1082 g Kaliumjodid (6,51 Mol) und 7000 ml H₂O vorgelegt. Bei 15 bis 20 °C wird innerhalb von 1 bis 2 Std. die Diazonium-Salzlösung zugefügt. Man verdünnt die erhaltene Suspension mit 10 Liter H₂O und saugt den Niederschlag ab. Dann wäscht man den Filterkuchen mit einer Mischung von 435 g Na₂S₂O₅ in 8,5 Liter H₂O jodfrei und wäscht mit 25 Liter H₂O neutral. Man erhält die Verbindung der Formel (IV) in einer Menge von 1771 g feucht. Nach Trocknen bei 50 C in Vakuum (= unter reduziertem Druck) liegen 1403 g (94,8 % d. Th.) 4-Jod-2-aminosulfonyl-benzoesäuremethylester (Sulfonamid der Formel IV) mit einem Schmelzpunkt von 175-177 °C vor.

### Beispiel 2

### 4-Jod-2-[N-(N-butyl-aminocarbonyl)-aminosulfonyl]-benzoesäuremethylester (I)

34,1 g (0,1 Mol) des Sulfonamids der Formel (IV) aus Beispiel 1, 14,7 g K₂CO₃ (0,105 Mol) werden mit 10,6 g n-Butylisocyanat (98 %ig) (0,105 Mol) in 250 ml Chlorbenzol versetzt und 2 Stunden bei 55 bis 60 °C gehalten. Anschließend rührt man 8,5 Std. bei 90 °C. Man kühlt auf Raumtemperatur, fügt zunächst 200 ml Wasser, dann 2n Salzsäure bis pH 1 bis 2 zu. Die organische Phase wird abgetrennt, viermal mit 50 ml Wasser säurefrei gewaschen. Nach Entfernen des Lösungsmittels im Vakuum verbleiben 43,8 g der gewünschten Verbindung der Formel (I) (99,3 % d. Th.) vom Schmelzpunkt 128 bis 130 °C.

### Vergleichsbeispiel (US-A-4566898)

Eine Mischung von 1063,4 g 2-(Aminosulfonyl)-4-chlorbenzoesäure-isopropylester, 590,4 g n-Butylisocyanat und 590,4 g K₂CO₃ in 10,8 Liter 2-Butanon werden über Nacht unter Rückfluß erhitzt. Nach Abkühlen auf Raumtemperatur gießt man die Reaktionsmischung in jeweils 10 Liter Eiswasser. Die wäßrige Phase wird mit 9 Liter Methylenchlorid extrahiert. Die wäßrige Phase wird mit konzentrierter HCI auf pH 1,0 angesäuert und der erhaltene Niederschlag filtriert. Nach Trocknung erhält man 714,6 g 2-[N-(N-Butyl-aminocarbonyl)-aminosulfonyl]-4-chlorbenzoesäureisopropylester (44 % d. Th.) vom Schmelzpunkt 129-132 °C.

## Patentansprüche

1. Verbindungen der Formel (I), 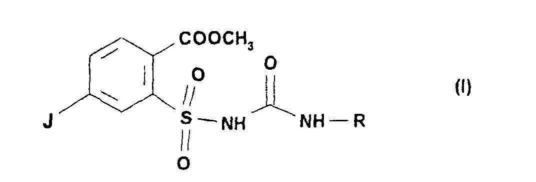 worin
R Alkyl oder Cycloalkyl, wobei jeder der beiden letztgenannten Reste unsubstituiert oder substituiert ist,
bedeutet.

2. Verbindungen nach Anspruch 1, **dadurch gekennzeichnet, daß**
R (C₁-C₁₂)Alkyl, das unsubstituiert oder durch einen oder mehrere Reste aus der Gruppe Halogen, (C₁-C₆)Alkoxy, (C₁-C₆)Alkylthio, (C₁-C₆)Haloalkoxy und gegebenenfalls substituiertes Phenyl substituiert ist, oder (C₃-C₁₂)Cycloalkyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₆)Alkylthio substituiert ist,
bedeutet.

3. Verbindungen nach Anspruch 1 oder 2, **dadurch gekennzeichnet, daß**
R (C₁-C₁₂)Alkyl, das unsubstituiert oder durch einen oder mehrere Reste aus der Gruppe Halogen, (C₁-C₄)Alkoxy und Phenyl substituiert ist, oder (C₃-C₁₂)Cycloalkyl
bedeutet.

4. Verbindung nach Anspruch 1, **dadurch gekennzeichnet, daß**
R n-Butyl bedeutet.

5. Verbindung der Formel (III), 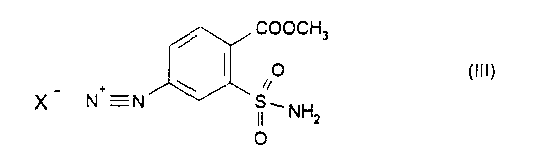 worin X⁻ das Äquivalent eines Anions bedeutet.

6. Verbindung nach Anspruch 5, dadruch gekennzeichnet, daß X⁻ = J⁻ bedeutet.

7. Verfahren zur Herstellung von Verbindungen der Formel (I), wie sie in Anspruch 1 definiert sind, **dadurch gekennzeichnet, daß** man eine Verbindung der Formel (IV) 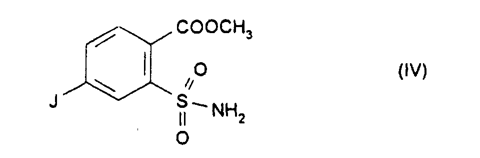 mit einem Isocyanat der Formel (V),
R-N=C=O (V)
worin R wie in Formel (I) definiert ist, zur Verbindung der Formel (I) umsetzt.

8. Verfahren nach Anspruch 7, **dadurch gekennzeichnet, daß** man
(a) eine Verbindung der Formel (II) 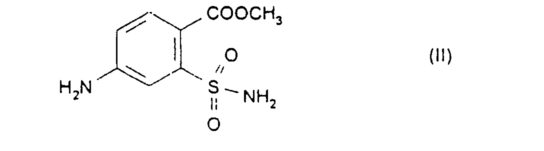 durch Diazotierung in Gegenwart einer Säure H⁺X⁻, wobei X⁻ ein Äquivalent eines Anions bedeutet, zu einer Verbindung (Diazoniumsalz) der Formel (III), 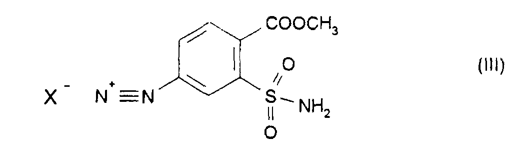 worin X⁻ die Bedeutung wie in der Säure H⁺X⁻ hat, umsetzt und
(b) die Verbindung der Formel (III) in Gegenwart von Jodidionen zu einer Verbindung der Formel (IV) 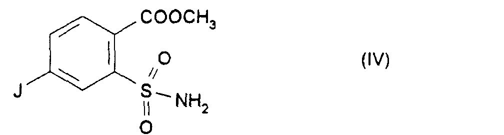 umsetzt und
(c) die Verbindung der Formel (IV) mit einem Isocyanat der Formel (V),
R-N=C=O (V)
worin R wie in Formel (I) definiert ist, zur Verbindung der Formel (I) umsetzt.

9. Verfahren zur Herstellung von Verbindungen der Formel (III), wie sie in Anspruch 5 oder 6 definiert sind, **dadurch gekennzeichnet, daß** man
(a) eine Verbindung der Formel (II) 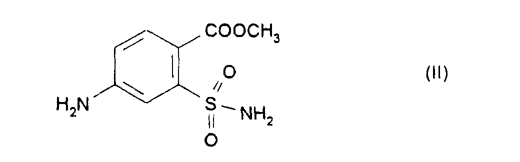 durch Diazotierung in Gegenwart einer Säure H⁺X⁻, wobei X⁻ ein Äquivalent eines Anions bedeutet, zur Verbindung (Diazoniumsalz) der Formel (III) umsetzt.

10. Verfahren zur Herstellung herbizider Sulfonylharnstoffe der Formel (I')
Aryl-SO₂-NH-CO-NH-R (I')
worin
Aryl 2-Carbomethoxy-5-iod-phen-1-yl und
R einen Rest der Formel 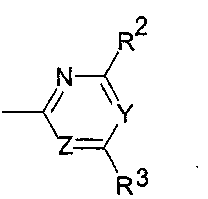
bedeuten, wobei
Y und Z unabhängig CH oder N, wobei Y und Z nicht gleichzeitig CH sind,
R² Wasserstoff, Halogen, (C₁-C₃)Alkyl, (C₁-C₃)Alkoxy, wobei die beiden letztgenannten Reste unsubstituiert oder ein- oder mehrfach durch Halogen oder (C₁-C₃)Alkoxy substituiert sind,
R³ Wasserstoff, Halogen, (C₁-C₃)Alkyl, (C₁-C₃)Alkoxy oder (C₁-C₃)Alkylthio, wobei die vorgenannten alkylhaltigen Reste unsubstituiert oder ein- oder mehrfach durch Halogen oder ein- oder zweifach durch (C₁-C₃)Alkoxy oder (C₁-C₃)Alkylthio substituiert sind, oder einen Rest der Formel NR⁵R⁶, (C₃-C₆)Cycloalkyl, (C₂-C₄)Alkenyl, (C₂-C₄)Alkinyl, (C₃-C₄)Alkenyloxy oder (C₃-C₆)Alkinyloxy und
R⁵ und R⁶ unabhängig voneinander Wasserstoff, (C₁-C₄)Alkyl, (C₂-C₄)Alkenyl, (C₁-C₄)Haloalkyl oder (C₁-C₄)Alkoxy
bedeuten,
**dadurch gekennzeichet**, dass man eine Verbindung der Formel (I), wie sie nach einem der Ansprüche 1 bis 4 definiert ist, mit Phosgen zu dem Isocyanat der Formel
Aryl-SO₂-NCO
umsetzt, welches anschließend mit einem Amin der Formel HN-R, worin R wie in Formel (I') definiert ist, zu dem herbiziden Sulfonylharnstoff der Formel (I') umgesetzt wird.

11. Verfahren nach Anspruch 10, **dadurch gekennzeichnet, dass** die Verbindung der Formel (I) unter Anwendung eines Verfahrens nach einem oder mehreren der Ansprüche 7 bis 9 hergestellt worden ist.

12. Verwendung von Verbindungen der Formel (I), wie sie gemäß einem der Ansprüche 1 bis 4 definiert sind, zur Herstellung herbizider Sulfonylharnstoffe.

## Claims

1. A compound of the formula (I), 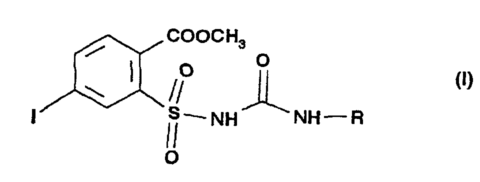 in which
R is alkyl or cycloalkyl, each of the last two radicals being unsubstituted or substituted.

2. A compound as claimed in claim 1, **characterized in that**
R is (C₁-C₁₂)alkyl which is unsubstituted or substituted by one or more radicals selected from the group consisting of halogen, (C₁-C₆)alkoxy, (C₁-C₆)alkylthio, (C₁-C₆)haloalkoxy and phenyl with or without substitution, or is (C₃-C₁₂)cycloalkyl which is unsubstituted or substituted by one or more radicals selected from the group consisting of halogen, (C₁-C₄)alkyl, (C₁-C₄)haloalkyl, (C₁-C₄)alkoxy, (C₁-C₄)haloalkoxy and (C₁-C₆)alkylthio.

3. A compound as claimed in claim 1 or 2, **characterized in that**
R is (C₁-C₁₂)alkyl which is unsubstituted or substituted by one or more radicals selected from the group consisting of halogen, (C₁-C₄)alkoxy and phenyl, or is (C₃-C₁₂)cycloalkyl.

4. A compound as claimed in claim 1, **characterized in that**
R is n-butyl.

5. A compound of the formula (III) 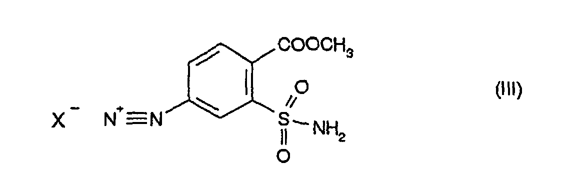 where X⁻ is the equivalent of an anion.

6. A compound as claimed in claim 5, **characterized in that** X⁻ = I⁻.

7. A process for preparing compounds of the formula (I) as defined in claim 1, **characterized by** reacting a compound of the formula (IV) 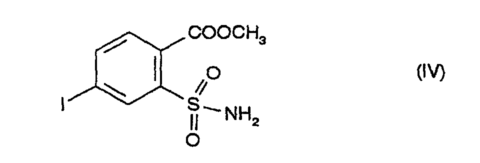 with an isocyanate of the formula (V)
R-N=C=O (V)
in which R is as defined in formula (I), to give the compound of the formula (I).

8. The process as claimed in claim 7, **characterized in that**
(a) a compound of the formula (II) 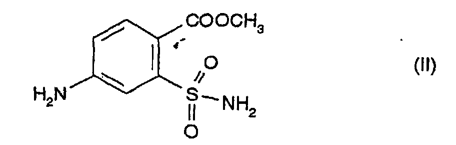 is diazotized in the presence of an acid H⁺X⁻, where X⁻ is an equivalent of an anion, to give a compound (diazonium salt) of the formula (III) 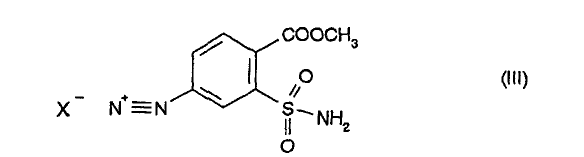 in which X⁻ has the same meaning as in the acid H⁺X⁻, and
(b) the compound of the formula (III) is reacted in the presence of iodide ions to give a compound of the formula (IV) 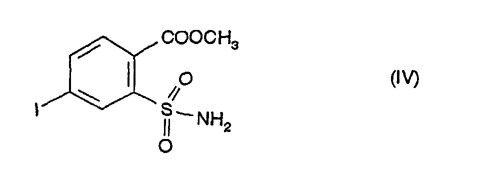 and
(c) the compound of the formula (IV) is reacted with an isocyanate of the formula (V)
R-N=C=O (V)
in which R is as defined in formula (I), to give the compound of the formula (I).

9. A process for preparing compounds of the formula (III) as defined in claim 5 or 6, **characterized in that** it comprises
(a) diazotizing a compound of the formula (II) 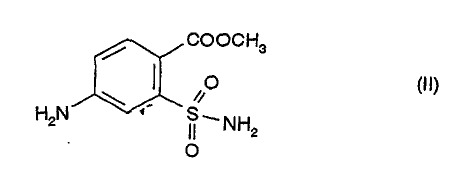 in the presence of an acid H⁺X⁻, where X⁻ is an equivalent of an anion, to give the compound (diazonium salt) of the formula (III).

10. A process for preparing herbicidal sulfonylureas of the formula (I')
Aryl-SO₂-NH-CO-NH-R (I')
where
Aryl is 2-carbomethoxy-5-iodophen-1-yl and
R is a radical of the formula 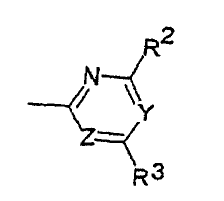
where
Y and Z are independently CH or N, although Y and Z must not be CH at the same time,
R² is hydrogen, halogen, (C₁-C₃)alkyl or (C₁-C₃)alkoxy, the last two radicals mentioned being unsubstituted or mono- or polysubstituted by halogen or (C₁-C₃)alkoxy,
R³ is hydrogen, halogen, (C₁-C₃)alkyl, (C₁-C₃)alkoxy or (C₁-C₃)alkylthio, the aforementioned alkyl-containing radicals being unsubstituted or mono- or polysubstituted by halogen or mono- or disubstituted by (C₁-C₃)alkoxy or (C₁-C₃)alkylthio, or is a radical of the formula NR⁵R⁶, (C₃-C₆)cycloalkyl, (C₂-C₄)alkenyl, (C₂-C₄)alkynyl, (C₃-C₄)alkenyloxy or (C₃-C₆)alkynyloxy and
R⁵ and R⁶ are independently hydrogen, (C₁-C₄)alkyl, (C₂-C₄)alkenyl, (C₁-C₄)haloalkyl or (C₁-C₄)alkoxy,
**characterized in that** a compound of the formula (I) as defined in any of claims 1 to 4 is reacted with phosgene to form the isocyanate of the formula
Aryl-SO₂-NCO
which is subsequently reacted with an amine of the formula HN-R, where R is as defined in the formula (I'), to form the herbicidal sulfonylurea of the formula (I').

11. The process as claimed in claim 10, **characterized in that** the compound of the formula (I) has been prepared using a process as claimed in one or more of claims 7 to 9.

12. The use of a compound of the formula (I) as defined in any of claims 1 to 4, for preparing herbicidal sulfonylureas.

## Revendications

1. Composés de formule (I), 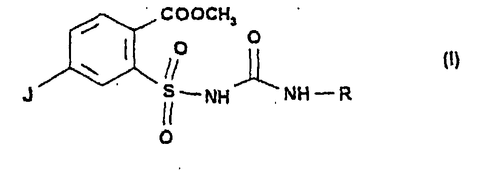 dans laquelle
R représente un groupe alkyle ou cycloalkyle, dans lequel chacun des deux derniers groupes mentionnés est non substitué ou substitué.

2. Composés selon la revendication 1, **caractérisés en ce que**
R représente un groupe alkyle en C₁ à C₁₂, qui est non substitué ou substitué par un ou plusieurs groupes choisis parmi un atome d'halogène, un groupe alcoxy en C₁ à C₆, alkylthio en C₁ à C₆, halogénoalcoxy en C₁ à C₆ et phényle éventuellement substitué, ou cycloalkyle en C₃ à C₁₂, qui est non substitué ou substitué par un ou plusieurs groupes choisis parmi un atome d'halogène, un groupe alkyle en C₁ à C₄, halogénoalkyle en C₁ à C₄, alcoxy en C₁ à C₄, halogénoalcoxy en C₁ à C₄ et alkylthio en C₁ à C₆.

3. Composés selon la revendication 1 ou 2, **caractérisés en ce que**
R représente un groupe alkyle en C₁ à C₁₂, qui est non substitué ou substitué par un ou plusieurs groupes choisis parmi un atome d'halogène, un groupe alcoxy en C₁ à C₄ et phényle, ou cycloalkyle en C₃ à C₁₂.

4. Composé selon la revendication 1, **caractérisé en ce que**
R représente le n-butyle.

5. Composé de formule (III), 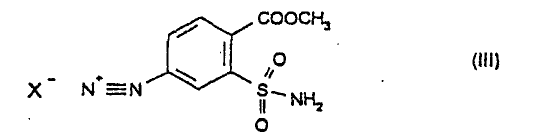 dans laquelle X⁻ représente l'équivalent d'un anion.

6. Composé selon la revendication 5, **caractérisé en ce que** X⁻ = I⁻.

7. Composé pour la préparation de composés de formule (I), comme ils sont définis à la revendication 1, **caractérisé en ce qu'**on met à réagir un composé de formule (IV) 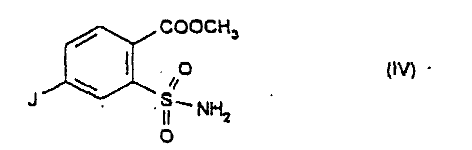 avec un isocyanate de formule (V),
**R-N=C=O (V)**
dans laquelle R est défini comme dans la formule (I), pour obtenir le composé de formule (I).

8. Procédé selon la revendication 7, **caractérisé en ce qu'**on met à réagir
(a) un composé de formule (II) 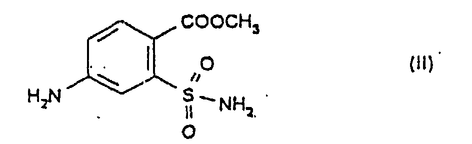 par diazotation en présence d'un acide H⁺X⁻, X⁻ représentant l'équivalent d'un anion, pour obtenir un composé (sel de diazonium) de formule (III), 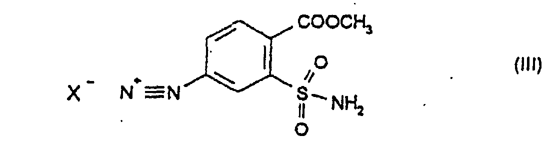 dans laquelle X⁻ a la même signification que dans l'acide H⁺X⁻, et on met à réagir
(b) le composé de formule (III) en présence d'ions iodure pour obtenir un composé de formule (IV) 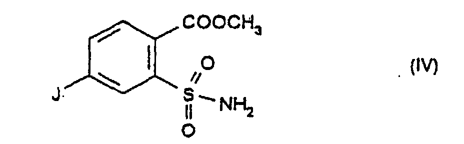 et on met à réagir
(c) le composé de formule (IV) avec un isocyanate de formule (V),
**R-N=C=O (V)**
dans laquelle R est défini comme dans la formule (I), pour obtenir le composé de formule (I).

9. Procédé pour la préparation de composés de formule (III), comme ils sont définis dans la revendication 5 ou 6, **caractérisé en ce qu'**on met à réagir
(a) un composé de formule (II) 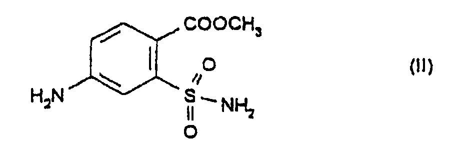 par diazotation en présence d'un acide H⁺X⁻, X⁻ représentant un équivalent d'un anion, pour obtenir un composé (sel de diazonium) de formule (III).

10. Procédé pour la préparation de sulfonylurées herbicides de formule (I')
**Aryl-SO**_{**2**}**-NH-CO-NH-R (I')**
dans laquelle
**Aryl** représente le 2-carbométhoxy-5-iodo-phén-1-yle et
**R** représente un groupe de formule 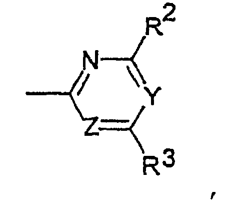
dans laquelle
Y et Z sont indépendamment CH ou N, Y et Z n'étant pas CH en même temps,
R² représente un atome d'hydrogène, d'halogène, un groupe alkyle en C₁ à C₃, alcoxy en C₁ à C₃, dans lequel les deux derniers groupes mentionnés sont non substitués ou substitués une ou plusieurs fois par un atome d'halogène ou un groupe alcoxy en C₁ à C₃,
R³ représente un atome d'hydrogène, d'halogène, un groupe alkyle en C₁ à C₃, alcoxy en C₁ à C₃ ou alkylthio en C₁ à C₃, dans lequel les groupes alkylés mentionnés précédemment sont non substitués ou substitués une ou plusieurs fois par un halogène ou une deux fois par un alcoxy en C₁ à C₃ ou un alkylthio en C₁ à C₃, ou un groupe de formule NR⁵R⁶, cycloalkyle en C₃ à C₆, alcényle en C₂ à C₄, alcynyle en C₂ à C₄, alcényloxy en C₃ à C₄ ou alcynyloxy en C₃ à C₆ et
R⁵ et R⁶ représentent indépendamment l'un de l'autre un atome d'hydrogène, un groupe alkyle en C₁ à C₄, alcényle en C₂ à C₄, halogénoalkyle en C₁ à C₄ ou alcoxy en C₁ à C₄,
**caractérisé en ce qu'**on met à réagir un composé de formule (I), comme il est défini selon une des revendications 1 à 4, avec du phosgène pour obtenir l'isocyanate de formule
**Aryl-SO**_{**2**}**-NCO,**
qui est ensuite mis à réagir avec une amine de formule HN-R, dans laquelle R est défini comme à la formule (I'), pour obtenir la sulfonylurée herbicide de formule (I').

11. Procédé selon la revendication 10, **caractérisé en ce que** le composé de formule (I) a été préparé en utilisant un procédé selon une ou plusieurs des revendications 7 à 9.

12. Utilisation de composés de formule (I), comme ils sont définis selon une des revendications 1 à 4, pour la préparation de sulfonylurées herbicides.
